(19) **Europäisches Patentamt / European Patent Office / Office européen des brevets**

(11) **EP 2 014 234 A1**

(12) **EUROPEAN PATENT APPLICATION**

(43) Date of publication:
**14.01.2009 Bulletin 2009/03**

(51) Int Cl.:
**A61B 7/00** *(2006.01)* **A61B 7/04** *(2006.01)*

(21) Application number: **07425340.2**

(22) Date of filing: **04.06.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **UNIVERSITA' DEGLI STUDI DI MILANO**
**20122 Milano (IT)**

(72) Inventors:
• **Riboldi, Stefano**
**20046 Biassono (Milano) (IT)**

• **Riva, Marco**
**24040 Canonica d'Adda (Bergamo) (IT)**
• **Spoletini, Enrico**
**20137 Milano (IT)**
• **Belloni, Federico**
**24050 Bariano (Bergamo) (IT)**

(74) Representative: **Deambrogi, Edgardo et al**
**Jacobacci & Partners S.p.A.**
**Corso Emilia, 8**
**10152 Torino (IT)**

(54) **Auscultation device with high ambient noise rejection and operating method thereof**

(57)     A device and a method for the auscultation of heart or lung sounds or similar acoustic signals generated by the internal organs of a body are described, based upon the acquisition of an auscultation signal including a useful signal superimposed on an ambient noise signal, and of an ambient noise signal nearby, and upon the rejection of the ambient noise signal component from the auscultation signal. The acquired signals are converted into digital electrical signals, and the target auscultation signal is obtained by subtraction of the ambient noise digital signal, filtered through a digital filter arrangement having an adaptive transfer function, from the digital auscultation signal. The filter coefficients of the adaptive digital filter arrangement are calculated according to the minimisation of the energy of the target auscultation signal, and more specifically according to the minimisation of an energy functional $\Phi^2$ defined as:

$$\Phi^2 = \sum_{n=-M}^{0} S(n)^2 + \sum_{k=0}^{N-1} X^2(k)$$

in which $S(n)$ is the target auscultation signal *and* $X(k)$ is the filter impulse response.

FIG.1

EP 2 014 234 A1

**Description**

The present invention concerns clinical auscultation devices that allow the auscultation of heart and lung sounds, and more specifically a digital auscultation device according to the preamble of claim 1 and an operating method of such a device according to claim 11.

Amongst medical devices known in the field that allow the auscultation of heart and lung sounds there are stethoscopes and phonendoscopes.

The stethoscope is an instrument consisting of an acoustic tube shaped like a cone the base of which, having a smaller diameter, is rested on the ear and the other end is rested on the patient. The stethoscope has the characteristic of allowing the auscultation of limited areas, but the amplification power is extremely low.

A phonendoscope is an instrument consisting of a bell shaped like a funnel closed in the part of greater diameter by an elastic membrane. The smallest diameter is connected to one or two tubes that convey the sound to the ears.

The improvement of the signal/noise, ratio represents one of the main problems in the field of treatment of the information that can be acquired from such conventional auscultation devices.

Currently there are complex electronic auscultation instruments, like digital stethoscopes, which make it possible to modulate the volume of the sound ausculted and allow the auscultation of heart and lung sounds with rejection of ambient noise.

Amongst these, the 3M Littmann 3000 model electronic stethoscope adopts a noise rejection technique directly at the acoustic level, through a specific configuration of the auscultation head, suitable for combining the wavefront of the ambient noise acquired through the patient's body together with the useful heart signal, with a second wavefront of just ambient noise, suitably delayed through the path of a different acoustic route, so as to be out of phase with each other and cancel each other out.

According to other known techniques, a suitable configuration of the plastic duct that guides the acoustic waves from the auscultation head up to the earphones allows the influence of the ambient noise to be minimized during the travel of the acoustic wave however without having an effect upon the ambient noise coming from the patient's body.

US 2004/0037429 describes an improved electronic stethoscope that adopts an active noise cancellation technique by destructive interference between an acquired signal, carrying the useful signal corrupted by the noise, and an ambient noise sample signal, however combining such signals in a less than optimal way.

The arrangement subject of US 2004/0037429 includes a first microphone adapted to be arranged in contact with the patient's body, which inevitably also captures the sounds generated in the surrounding area, and a second microphone, arranged close to the first, adapted to record the ambient noise only.

The outputs of both the microphones are connected to a microprocessor arranged to carry out a spectral subtraction algorithm, adapted to produce a noise cancellation signal based upon the signal acquired by the second microphone, phased out by 180 degrees and suitably amplified to correspond to the ambient noise collected by the first microphone and cancel out its contribution.

All of the known solutions suffer overall from a high residual sensitivity to ambient noise, since they do not make it possible to calibrate the ambient noise suppression method according to the ambient variables and they do not have heart signal analysis technology.

Even the most advanced solutions, for example the one described in US 2004/0037429, which exploit spectral noise subtraction algorithms, are intrinsically limited in application by a priori assumptions on the nature of the noise (for example, that it is stationary over time) and on the time interval of useful signal (for example, in the case of the heart beat signal, limited to systole and diastole events), which jeopardise its effective practical use.

The purpose of the present invention is to provide an electronic auscultation device with improved behaviour with regard to the rejection of ambient noise, the effectiveness of which is not limited by the different possible ambient conditions, and also adapted for use in high-noise conditions, typical of use outside of hospital buildings and casualty departments like accident sites or emergency vehicles.

A further purpose of the invention is to provide an auscultation device adapted to be used by people with hearing difficulties.

Yet another purpose is to provide an auscultation device that offers advanced possibilities of interfacing with one or more users.

According to the present invention, such objects are accomplished thanks to a digital auscultation device having the characteristics defined in claim 1, and an operating method of such a device having the characteristics outlined in claim 11.

Particular embodiments are the subject of the dependent claims.

In summary, the device according to the invention is a device for the auscultation, processing, transmission and storage of heart and lung sounds.

The invention is based upon the use of a signal processing technique for rejection of the ambient noise based upon the two-channel acquisition of the sound and the subsequent reduction of the ambient noise through the use of digital filters with adaptive filtering algorithms, based upon the principle of correlation/perpendicularity of signals. Such algorithms, whilst being relatively more complex in terms of computing resources, are intrinsically superior in terms of performance compared to known spectral noise subtraction algorithms.

The device object of the invention also preferably has a plurality of audio, video and data interface functions, which make it possible for it to be used remotely, allowing remote diagnosis, particularly useful in areas in which the density of specialized medical staff is not high, as well as the possibility of storing the acquired signals and evaluating their evolution over time. The device again preferably has a frequency signal equalisation circuit, so as to make it possible for it to be used by people with different hearing abilities.

These characteristics are linked to the digital nature of the signals processed by the device, as indeed it is not possible to take advantage of them through a classic conventional stethoscope.

The possibility of ambient noise rejection is made possible by means of the presence of a pair of microphone transducers arranged at the auscultation head of the device and the use of advanced filtering techniques. A first microphone, arranged inside the head or auscultation chamber, is adapted to collect the useful signal and the possible ambient noise superimposed on it; a second microphone, arranged outside of the acoustic duct connected to the auscultation head, is adapted to capture, to a first approximation, the ambient noise only.

An adaptive digital filter calculated in real time is arranged to mix together the two signals and achieve the minimisation of the energy of the signal resulting from the combination of the two original signals, as a result supplying a signal in which, to a first approximation, just the acoustic contributions of the two original signals remain, which are not mutually correlated, therefore reducing the disturbance effect caused by the ambient noise and substantially improving the objective and subjective quality of the auscultation.

The digital filter synthesised through the adaptive digital algorithm does not depend at a first degree upon the nature of the ambient noise and thus maintains its effectiveness even in the presence of highly unstationary noise. Instead, it takes into account the operating conditions of the device (configuration of the patient's body and pressure level of the head on it, spatial distribution and possible movement of the ambient noise sources), adapting itself in real time.

The device according to the invention therefore has high immunity to ambient noise and automatic adaptation features not only with respect to the operating conditions relative to the location (reverberation, echo, spatial distribution of the noise sources), but also with respect to its coupling with the individual patient under examination (for example, different according to the characteristics of the thoracic cavity and consistency of the adipose masses in-between).

These features make it possible for it to be used in acoustically unfavourable locations and locations that greatly differ from each other.

From the useful signal, purified from the contribution of ambient noise of the first order and in digital format, the device is adapted to generate its corresponding reply in analogue form, which can be made directly available through acoustic transducers (headphones, loudspeakers, etc.) to address the needs of use by users without serious hearing difficulties, or to carry out a frequency equalisation of the signal so as to compensate "the acoustic capabilities" of the user and allow the device to also be used by subjects suffering from slight hearing difficulties.

This last step of conversion into an analogue signal of the ausculted sound can be further completed by taking into account the contribution of ambient noise present in the user's auditory canal, thus providing a signal that is further corrected so as to cancel out this additional component.

Regarding this, it should be observed that the noise component perceived inside the human ear is different to that present on the auscultation head of the device, because it is influenced by the different acoustic sound propagation pathways, and the aforementioned cancellation operation can be carried out by using suitable active headphones with high immunity to ambient noise or through making headphones with dedicated configuration.

Finally, the device has interface functions adapted to provide the information (the signals) acquired according to different modes of representation, for example providing such information through a visual channel, i.e. showing the progression over time of the signals through a screen arranged on the body of the instrument or a display of external units such as a PC monitor, or a PDA or telephone apparatus display, by using for example display techniques typical of digital oscilloscopes (trigger, image persistence, overall average, etc.).

Advantageously, this feature of the device makes it possible to respond to the needs of those, in the medical profession, who need to use the stethoscope, whilst not being able to make use of the listening apparatus to make a diagnosis, for example because seriously or totally hard of hearing.

In addition to this, a further important feature consists of the possibility of storing the collected signal data for later off-line analysis, for example in order to break down the fundamental characteristic parameters of the signals acquired, to highlight the presence of elements worthy of deeper investigation (heart beat regularity and frequency, presence of possible "murmurs", anomalous tones in addition to the two main tones, etc.).

Lastly, the availability of good quality signals, directly available in digital format in the form of numerical files inside a processing system, makes it possible to transfer the sounds onto memory supports and allows the wave shapes of interest to be filed in databases, so as to allow, for example, a later comparison of the different signals of the same patient collected over time.

Further advantages and characteristics of the present invention shall become clearer from the following detailed description, made with reference to the attached drawings, provided as a non-limiting example, in which:

figure 1 is an overall schematic representation of an auscultation device according to the invention;
figure 2 is a depiction of use of the auscultation device of figure 1;
figure 3 is a block circuit diagram of an electronic arrangement for processing the acoustic signals acquired by the device according to the invention;
figures 4a-4d, 5a-5d, 6, 7 are graphs indicative of the outcomes of acquisition and processing signals, respectively in a first low ambient noise condition and in a second high ambient noise condition, and relative characteristic responses of the digital filter.

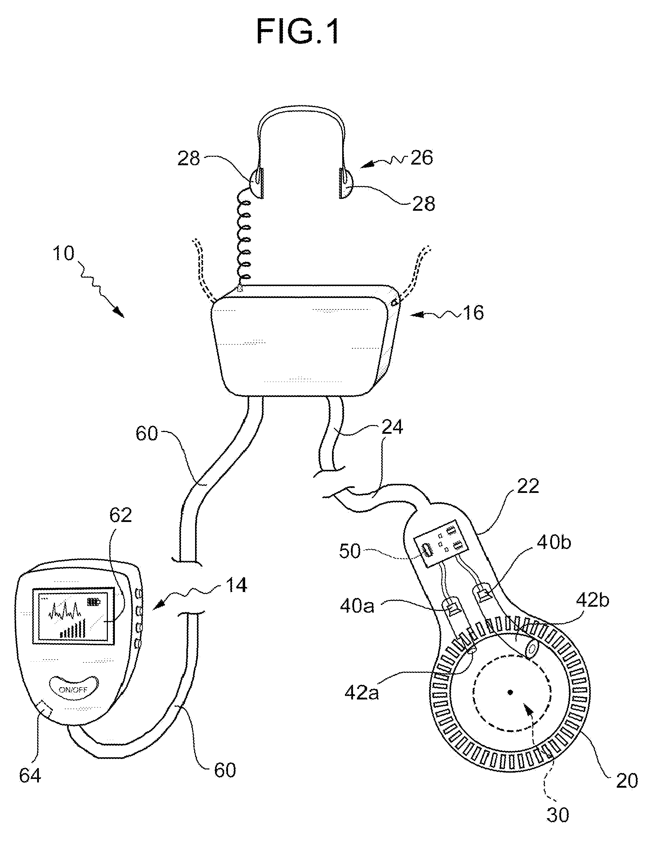

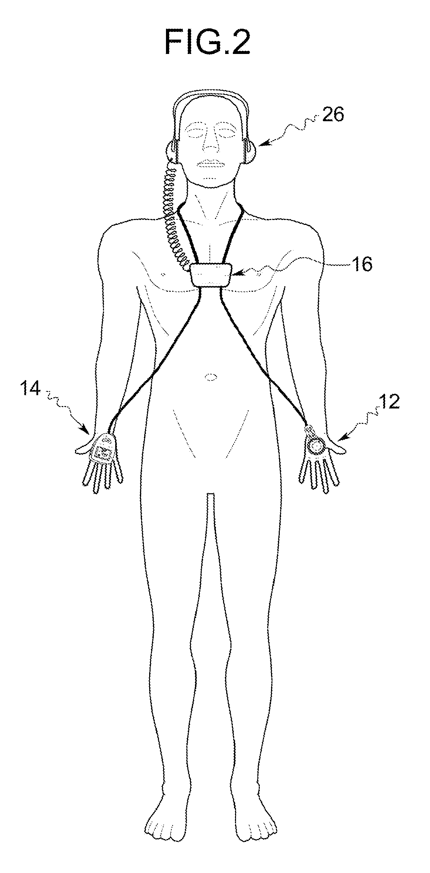

With reference to figures 1 and 2 an auscultation apparatus 10 according to the invention is shown, comprising an auscultation device 12, a processing unit provided with display means 14 and a battery power supply device 16.

The auscultation device 12 comprises an auscultation capsule 20 formed in a main body 22, that a cable 24 places in communication with the processing unit 14 and a headset 26, connected to the unit 14, provided with a pair of earphones 28 with low noise content, like those currently available on the market for high-fidelity musical applications.

The capsule comprises a chamber 30 for collecting acoustic signals, intended to be applied to the body to be ausculted and adapted to collect the acoustic signals generated by the inner members of such a body.

The chamber 30 is shaped according to the prior art and shall not be described further at this stage.

The main body of the capsule 20 houses a pair of microphones 40a, 40b adapted to convert the collected acoustic signal into an electrical signal, for subsequent processing.

The microphones are, for example, capacitor microphones, and are exposed to the sound field through a respective tubular collection duct 42a, 42b. A first microphone 40a is placed in communication with the auscultation chamber 30 to collect the useful signal and the possible superimposed ambient noise, whereas the second microphone 40b is placed in communication with a region outside the auscultation chamber and adjacent to it, and therefore it is adapted to capture, to a first approximation, the ambient noise only.

Downstream of the microphones an electronic amplification and filtering circuit 50 for processing the detected signals is inserted into the body of the capsule 20.

A D/A converter performs the conversion of the processed numeric signal into an analogue signal for its diffusion, through the headset 26, to the user's ear.

The display device 14, connected through a relative cable 60 to the power supply device 16, comprises a screen 62 for representing the signals acquired according to different ways, for example showing the progression over time of the signals through a screen arranged on the body of the instrument, for example using the display techniques typical of digital oscilloscopes (trigger, image persistence, overall average, etc.) and connection means such as a USB 64 port or wireless interfaces for data transfer to external unit displays, such as a PC monitor, a PDA or telephone apparatus display and similar. In addition to this, the device 14 includes storage modules (not shown) adapted to store the data, in digital format, of the collected signals for them to be subsequently analysed off-line, for example for their transferral onto external storage supports again through the USB 64 port.

Advantageously, the device 14 processes the signals received by the circuit 50 incorporated in the auscultation device through the cable connections 24 and 60.

The operation of the digital electronic stethoscope can occur in two ways: stand-alone or in wireless connection (WiFi or Bluetooth) with a PC desktop, palmtop PC (PDA), and/or even with latest generation mobile phones. In stand-alone mode auscultation, volume adjustment, detection of the main parameters of the heart signal and temporary storage of the heart signal are made possible. The information is supplied to the user through the screen. In remote wireless connection, a suitable interface programme makes it possible to manage a patient database, recording all of the auscultations and to display the relative phono-cardiograms, so as to make it possible, by comparison (audio and/or video) of successive auscultations over time, to track the progression of a pathology.

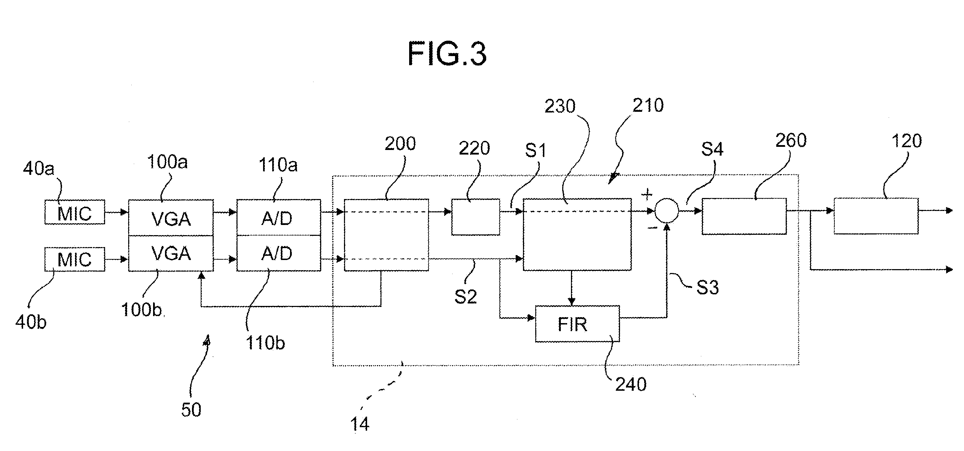

Figure 3 shows a block circuit diagram of an electronic arrangement for processing the acoustic signals acquired by the device according to the invention.

Such an arrangement can, for example, be made in the form of a circuit board based upon DSP or FPGA technology.

As stated above, the microphone indicated with 40a is adapted to collect the useful signal and the possible superimposed ambient noise, whereas the microphone indicated with 40b is adapted to capture the ambient noise only. The electrical outputs of the microphones 40a, 40b are connected to respective variable gain amplifying circuits 100a, 100b with integrated low-pass filter, downstream of which corresponding analogue/digital converters 110a, 110b are arranged.

The processing and filtering circuit 14 receives in input the outputs of the A/D converters, performs the filtering of the noise signal and the frequency equalisation, providing a processed auscultation signal, for use in digital data format in view of subsequent storage, display or transmission operations, or routing to a digital/analogue converter 120 for conversion into an analogue electrical signal in view of feeding an electro-acoustic converter circuit for transduction into an acoustic signal for direct auscultation.

Filtering occurs through implementation of an adaptive FIR digital filter, which shall be illustrated in detail in the remaining of the present description.

The processing and filtering circuit 14 includes, at its input, a circuit module 200 for automatically controlling the gain of the amplifiers 100a, 100b, adapted to measure the amplitude (the value) of the signals at its inputs, the control output of which is provided in feedback to said amplifiers.

The module 200 is substantially transparent to the signals that it receives in input, apart from adjusting their amplitude, through feedback towards the blocks 100a and 100b. The signals are provided downstream to a filter arrangement 210, respectively indicated S1 (the useful signal with superimposed ambient noise) and S2 (the signal with ambient noise only). Preferably, the signal S 1 is provided in input to the filter arrangement 210 after passing a delay block 220.

The filter arrangement comprises a module 230 for calculating the optimum coefficients of an FIR filter, adaptable to receive in input the signals S 1 and S2, and an FIR digital filter module 240, which receives, at a data input, the signal to be filtered S2 and, at a control input, the updated filter coefficients for the adaptation of the filter to the current signal.

The filter module 240 emits in output an adapted noise signal S3, subsequently subtracted from the signal S1 to form a pure auscultation signal S4. Downstream a frequency equalising circuit 260 is arranged that is adapted to perform a frequency equalisation of the acquired signal, before the emission of a processed auscultation signal for use as digital datum or for routing to the digital/analogue converter 120.

Again with reference to figure 3 the operation of the adaptive digital filter for the cancellation of the ambient noise is illustrated hereafter.

The microphone 40a acquires the useful signal (for example, the heart signal) superimposed to the ambient noise, which reaches it crossing the patient's body and the auscultation chamber (head).

The microphone 40b acquires the ambient noise only, directly or in any case detected through a transmissive means different from the previous one.

It is inevitable that the ambient noise is thus perceived differently at the two microphones.

From the conceptual point of view, the operation that one wishes to carry out to eliminate the noise contribution from the acquisition of the microphone 40a consists, in principle, of subtracting the signal S2 of the microphone 40b (noise) from the signal S1 of the microphone 40a (useful signal + noise).

From the operative point of view this simple process is, however, of no interest, since it is based upon the assumption that the ambient noise acquired by the two channels is the same, and this is not the case.

It is worth highlighting that the acoustic properties of the patient's body can also be highly variable from one time to the next (for example according to the body mass index, or the size of the thorax) and that the perception of the ambient noise through the head of the stethoscope varies considerably, also according to the contact pressure between this and the patient's body.

Another fundamentally important factor is given by the fact that the acoustic noise wave, generated, for example, by a spatially concentrated source, propagating in the air at finite speed, can never reach the two microphones at the same moment in time, since they are physically arranged in two different places, although close to one another.

In a more realistic case with a plurality of ambient noise sources, or else in conditions of reverberation of the walls of a room in which auscultation takes place, all of the ambient noise contributions would thus be delayed or arrive early differently between the two microphones.

This physical variability must then inevitably be added to with the variability in the translation process from acoustic signal to electrical signal, which is particular to each individual microphone and that, once a pair of microphones has been fixed, can again depend upon physical factors such as temperature and humidity of the surrounding area.

Lastly, the analogue amplification circuits (VGA) and the analogue/digital converters themselves also introduce a slight difference in magnitude between two sampled signals S1 and S2.

Faced with such a complicated situation, the function of the FIR filter applied to the signal S2 is that of generating in output from it a signal S3 in which the noise components are exactly, or in any case as close as possible to, those superimposed to the heart beat and present in the signal S1.

The design of the filter arrangement is based upon two basic assumptions.

The first assumption is that the useful signal is not practically acquired by the microphone 40b, which indeed has no voluntary acoustic connection with the auscultation head, whilst being arranged close to the patient's body.

Experience demonstrates that the magnitude of the heart beat perceived a few centimetres from a patient's body is negligible, if not zero. As testimony of this we have calculated such a contribution in the signal of the microphone 40b through an analysis of the mutual correlation function between the two signals S 1 and S2 in the absence of ambient noise, which proved to be practically zero.

The second basic assumption consists of hypothesising a linear behaviour of the entire analogue signal acquisition chain, starting from the microphones up to the analogue/digital converters.

In order to make such a hypothesis realistic, it is advantageous to take maximum care in selecting the microphones that, whilst being extremely small in size (3 x 3 x 2 mm$^3$), must have very good performance also in terms of linearity of the response, like for example the microphones that can be used in high-level hearing aids or acoustic prostheses. The imperfect linearity of the response of the microphones is inevitably the aspect that determines the final limit to the performance of the system.

The analogue processing electronics (low-noise amplifiers and low-pass filters) must preferably be made with feedback circuits, the linearity error of which is limited, for example guaranteed to be less than one part in 10000.

Subject to these two fundamental hypotheses, the synthesis technique of the FIR filter is based upon the principle of minimisation of the energy of the signal S4, i.e. the pure auscultation signal sought after, difference between S and S3, the calculation of which is carried out in module 230 for calculating the optimum coefficients of the filter, based upon the input signals S1 and S2.

Based upon the second hypothesis, the signal S1 can be expressed as the sum of two components independent from one another (thus statistically perpendicular to one another with respect to the scalar product between sampled signals), the first linked to the ambient noise and the second to the useful signal. Such components, whilst always being physically independent at the acoustic wave level, maintain such independence in the signal S 1 just in the hypothesis of linear analogue processing.

In this hypothesis, the energy of the signal S 1 thus coincides with the sum of the energies of its two noise and useful signal components, which at this level are mixed together and not immediately separable.

The signals S2 and S3 consist of the ambient noise contribution only.

The energy of the signal S4, difference between S1 and S3, can be expressed in the same way as the sum of two components, the first associated with the useful signal and the second associated with the ambient noise, the latter only dependent upon the properties of the predetermined FIR filter (based upon the first fundamental hypothesis for which the FIR filter acts exclusively on the ambient noise component).

The FIR filter that minimises the magnitude of the energy of the signal S4, thus only intervenes on the energy of the ambient noise component contained in S4 and not on the component linked to the useful signal in S4 and - in optimal conditions - cancels out the energy of such a noise component. In such an operative condition, to be achieved with an adaptive calibration step of the duration of a fraction of a second, the signal S4 thus contains just and precisely the heart beat component contained in S1, but no longer the noise that was superimposed to it in S1.

It should be noted that the adaptive FIR filter that minimises the energy of the signal S4 can never in theory manage to cancel out such energy, but just to reduce it to a minimum value. Such a minimum value corresponds exactly to the energy of the useful signal present in the signal S4, which is statistically perpendicular to any variation that the FIR filter can make with respect to the signal S4, since it does not operate on the useful signal (first hypothesis) and since useful signal and ambient noise are independent from each other (second hypothesis).

The useful consequence of operating in these conditions is that the minimum possible energy signal S4 corresponds, in principle, to the useful signal, i.e. with the only signal that cannot be eliminated from S4 because it is present in only one of the two acquisition channels.

It is important to note that this calibration method of the FIR filter can operate in real time during the auscultation process of the patient by the doctor, thus also in the presence of a heart beat or lung respiration signal and that, since no preliminary calibration process is necessary, the use of the digital stethoscope is totally similar to that of a conventional stethoscope.

The numerical technique used to minimise the energy functional of S4, based upon which the module 230 operates, is briefly recalled here.

The numerical expressions for the signals S3 and S4 are obtained as a function of the signals S1, S2 and of the unknown coefficients of the FIR filter [x(1), x(2), ..., x(N)], where N is the order of the filter, based upon the fundamental theorem of convolution of linear systems.

$$S3(n) = \sum_{k=0}^{N-1} S2(n-k) \cdot X(k)$$

where *X(k)* is the impulse response of the filter, and

$$S4(n) = S1(n) - S3(n) = S1(n) - \sum_{k=0}^{N-1} S2(n-k) \cdot X(k)$$

The resulting energy of the signal S4 is therefore:

$$\sum_{n=-M}^{0} S4(n)^2 = \sum_{n=-M}^{0} \left( S1(n) - S3(n) \right)^2 = \sum_{n=-M}^{0} \left( S1(n) - \sum_{k=0}^{N-1} S2(n-k) \cdot X(k) \right)^2$$

The minimisation of the previous expression as a function of the unknown coefficients $X(k)$ defining the FIR filter, whilst precisely meeting the requirement of minimisation of the energy of the signal S4, determines in general filters that are not totally satisfactory from the application point of view. In particular, the coefficients $X(k)$ frequently change sign and maintain high values in the module. Such a condition is disadvantageous from two points of view: firstly, the effect of the quantizing noise introduced by the ADC 110b that converts the signal of the ambient noise only is substantially increased at the output of the filter itself, based upon the following relationship:

$$E[OutNoise^2] = E[InNoise^2] \cdot \sum_{k=0}^{N-1} X^2(k)$$

Moreover, since the coefficients $X(k)$ have a high absolute value, their implementation in fixed point processors requires a large number of bits.
In order to synthesise an FIR filter with good properties of treatment of the quantizing noise of the sampled signals, and whose coefficients can be expressed through a physical embodiment with a small number of bits, it is thus suitable to modify the expression to be minimised, adding a further term to it and reaching the functional $\Phi^2$ :

$$\Phi^2 = \sum_{n=-M}^{0} S4(n)^2 + \alpha \cdot \sum_{k=0}^{N-1} X^2(k)$$

In this way, the synthetised FIR filter, whilst still meeting the fundamental requirement of minimising the energy of the signal S4, also simultaneously has good properties with respect to both of the problems listed above, since its numerical coefficients $X(k)$ are intrinsically aimed at not pointlessly taking on a large absolute value.
The value of the weight coefficient $\alpha$ is determined automatically at the moment of synthesis of the FIR filter $X(k)$ according to the procedure described hereafter.
The value of the functional $\Phi^2$, which has the characteristics of a quadratic form defined as positive, by definition has one and only one global minimum as a function of the N unknowns $X(k)$ representing the FIR filter to be determined.
By calculating all of the N partial derivatives of the functional with respect to each unknown $X(k)$ one obtains a linear system of N equations in N unknowns, which can be solved through standard matrix inversion techniques.
The expression in matrix form of such a system can be generically indicated as:

$$A \cdot X = B$$

The data matrix A, of size N x N, is updated in real time each time a new piece of sampled data of the ambient noise signal is available to the processing system 14. In particular, downstream of the relative calculations, one reaches a simple expression for updating the matrix A, relative to the first term of the functional. Since the two vectors containing the last N samples of the signal S2 are of the column and row type respectively, the expression is:

$$A(n) = A(n-1) +$$
$$+ [S2(n) \quad S2(n-1) \quad ... \quad S2(n-N+1)] \cdot [S2(n) \quad S2(n-1) \quad ... \quad S2(n-N+1)]'$$

Once a sufficient number of samples has been collected, indicatively equal to about 10,000, the corresponding FIR filter $X(k)$ is synthesised. Before this it is, however, necessary to further modify the data matrix A, in order to also consider the second part of the functional. For this purpose it is sufficient to add a further diagonal matrix whose coefficients are equal in value to the parameter $\alpha$ to the matrix A defined up to now. Such a parameter can be empirically, but effectively determined by considering the average value of the coefficients on the main diagonal of the original matrix A and dividing such a value by 100,000. From this it can be seen that the perturbation introduced by the second regulating term into the functional to be minimised is negligible with respect to the contribution of the first fundamental term, such that the synthesised FIR filter still fully satisfies the requirement of minimisation of the energy of the signal S4, in addition having the aforementioned characteristics that improve its performance with respect to the quantizing noise of the A/D converter and that make it easier to implement in fixed point calculation devices.

The matrix A of the data to be inverted, which by its nature is already symmetrical, but of substantial size (for example, 200 x 200 elements) can be directly inverted, or else transformed in advance towards a Toeplitz matrix so as to make the subsequent inversion operation easier.

Each calculation operation of the coefficients of the FIR filter requires the availability of signal acquisitions lasting about 250 ms, so as to be able to best adapt the filtering by a number of 3-4 times per second.

The adaptation of the filter can advantageously be suspended in the case in which the form of the FIR filter calculated is much different to the form being used previously, this being an event that can be referred to transitory anomalous situations, like for example the doctor accidentally striking the head with his hand.

Finally, it should be noted that the presence of the delay block 220 on the line of the signal S 1 is important to make it physically possible to synthesise the correct FIR filter. In general, the wavefront of each ambient noise source can reach the two microphones with a relative positive or negative delay, according to the location of the noise source with respect to the head of the stethoscope and the spatial orientation thereof. Since the speed of the acoustic wavefront is equal to about 340 metres per second in air and the maximum difference in the two paths is less than 5-6 cm, the time delay is always less than 0.2 ms as an absolute value. Faced with a sampling frequency equal, for example, to 40 kHz, such a time delay means a delay of 8 digital samples. If the delayed signal is S 1 relative to the heart beat, the optimum FIR filter, synthesised by minimising the functional $\Phi^2$, automatically compensates such a delay introducing a corresponding delay in the path of the signal S2 on which it operates. If, vice-versa, the delayed acoustic signal were the one relative to S2, the FIR filter could not compensate such a delay, since it would absurdly have to be anti-causal in nature and precede the signal S2, a thing that is obviously not physically possible. The function of the delay block 220 is therefore to ensure that in all operating conditions and for any distribution of the ambient noise sources, the signal S 1 never arrives prior to the signal S2. Based upon what has been outlined above, a digital delay line equal to 10 samples achieves what is required, without moreover appreciably penalising auscultation since the according delay of 0.2 ms is absolutely negligible to the user.

Hereafter some results obtained at the testing stage are shown.

Two different ambient situations are presented, respectively with low ambient noise content (for example, the noise that can be captured in a street-front laboratory, not insulated from noises outside) and high ambient noise content (for example, the natural ambient noise of the previous case with addition of a concentrated source of high monochromatic artificial noise, purposefully introduced).

Hereafter it can be demonstrated that the effectiveness of the device according to the invention does not depend upon the specific nature of the ambient noise and the selection of a monochromatic artificial noise component is simply linked to a more obvious visual interpretation of the results.

CASE 1: Auscultation of the heart beat in a situation of low ambient noise

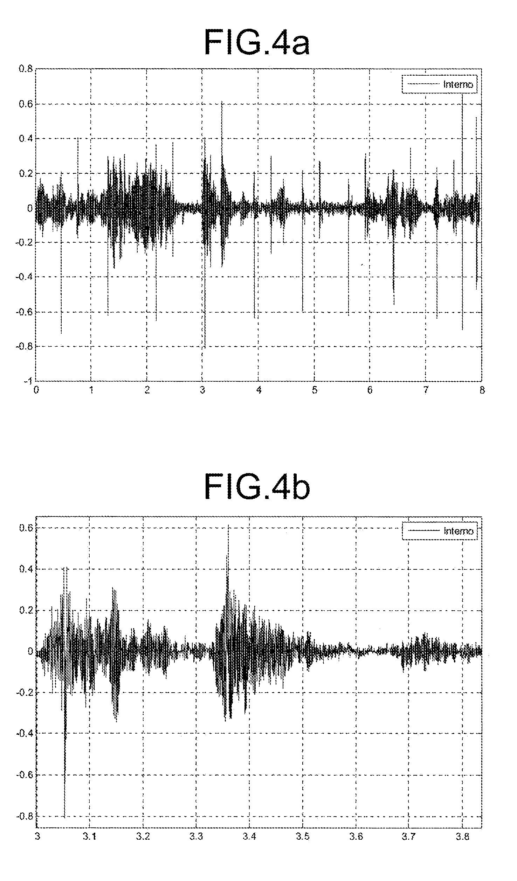

Figure 4a shows the progression of the signal acquired by the microphone inside the auscultation capsule, including a useful signal component (the heart signal) and an ambient noise signal component.

Figure 4b shows a time scale range of the signal of figure 4a, from which it is clear how the ambient noise partially masks the heart signal.

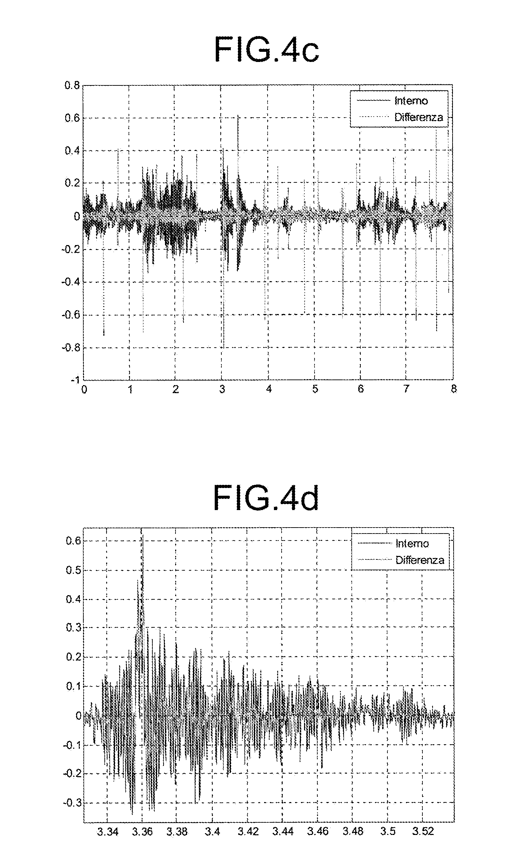

Figure 4c in comparison shows the effect of the filter algorithm on the heart signal disturbed by ambient noise: the overall signal made up of useful signal and noise is shown in dark grey tone, whereas the useful signal purified of the noise component is shown in light grey tone.

Figure 4d shows a time scale range of the signal of figure 4c in which it is possible to note the improvement of the signal/noise ratio compared to what is shown in Figure 4b.

CASE 2: Auscultation of heart beat in the case of high ambient noise

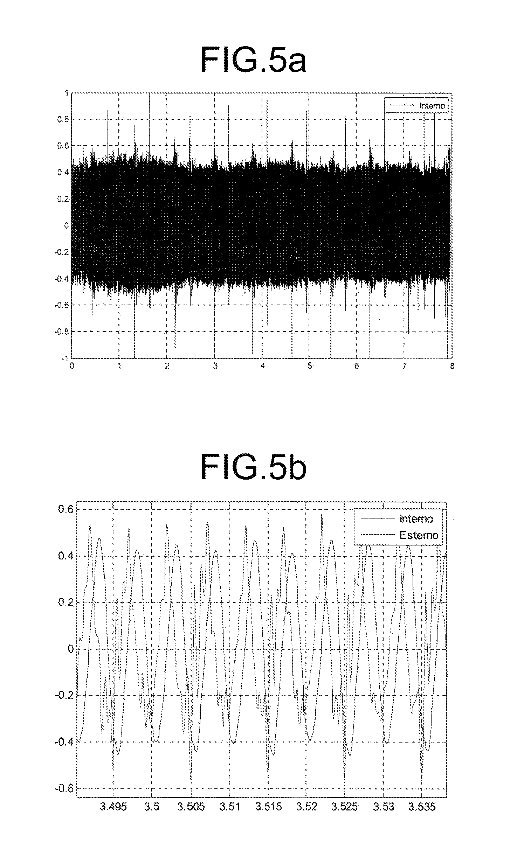

Figure 5a shows the evolution of the signal acquired by the microphone inside the auscultation capsule, including a useful signal component (the heart signal) and an ambient noise signal component, in the case of a particularly noisy location.

Figure 5b shows a time scale range of the signal of figure 5a, from which it is clear that the signal/noise ratio has a low value (almost unitary), and at the same time that the evolution over time of the two signals is different, such as to make a simple subtraction technique ineffective.

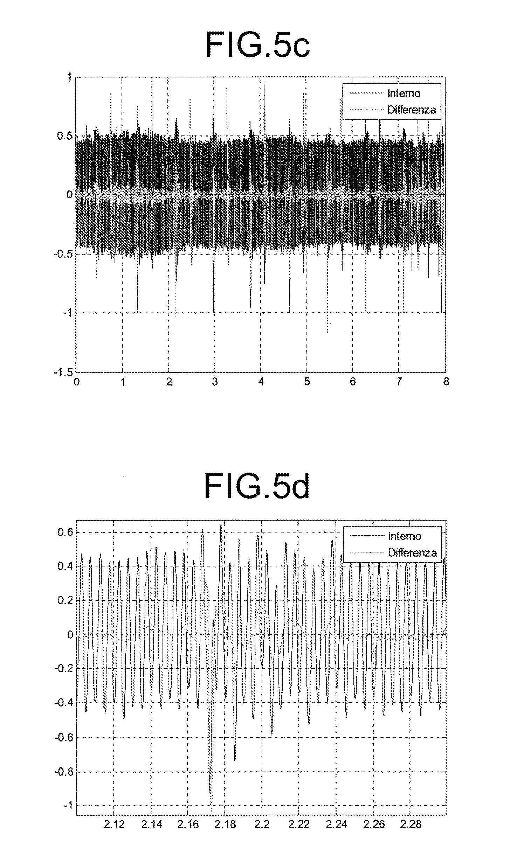

Figure 5c in comparison shows the effect of the filter algorithm on the heart signal disturbed by ambient noise: the overall signal made up of useful signal and noise is shown in dark grey tone, whereas the useful signal purified of the noise component is shown in light grey tone.

Figure 5d shows a time scale range of the signal of figure 5c in which it is possible to note the improvement of the signal/noise ratio compared to what is shown in Figure 5b.

The device according to the invention manages to obtain the results shown through an accurate calculation of the optimum filter most suitable for amplifying the heart signal according to the ambient characteristics, and it is thus characterized as a completely automatic adaptive system.

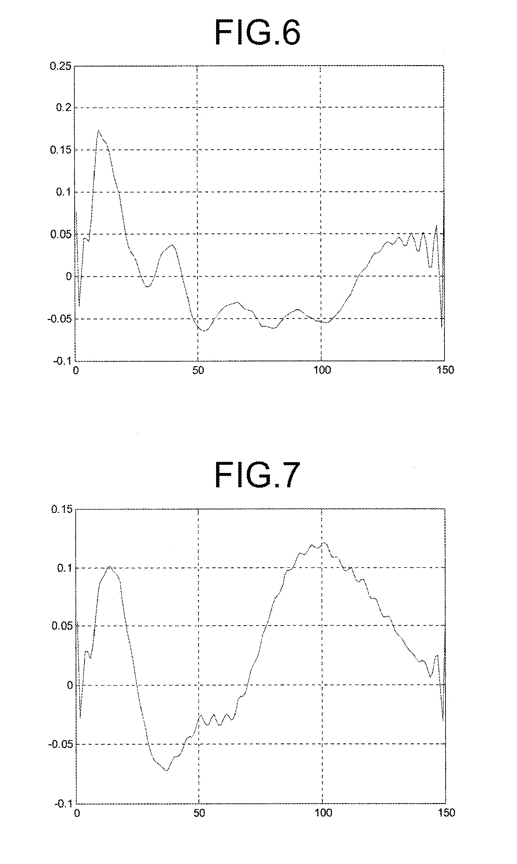

For the examined cases the relative characteristic responses of the filters used are respectively shown in figures 6 and 7. The partial difference between the two filters synthesised by the same adaptive algorithm is linked firstly to the addition of a new spatial source of concentrated noise (close to the patient's body) and, secondly, to the inevitable variation in the operating conditions of the instrument (position and pressure of the auscultation head, etc.).

Of course, without changing the principle of the invention, the embodiments and the details can be widely varied with respect to what has been described and illustrated purely as a non-limiting example, without for this reason departing from the scope of protection of the present invention defined by the attached claims.

**Claims**

1. Auscultation device of heart or lung sounds or similar acoustic signals generated by the internal organs of a body, comprising:

   - first acousto-electrical transducer means adapted to detect, through a first acoustic channel, an auscultation signal including a useful signal superimposed to an ambient noise signal, and generate a first analogue electrical signal indicative of said auscultation signal;
   - second acousto-electrical transducer means arranged in proximity to said first transducer means, adapted to detect, through a second acoustic channel, solely an ambient noise signal, and generate a second analogue electrical signal indicative of said ambient noise signal; and
   - processing means arranged to reject the ambient noise signal component from said auscultation signal,

   **characterised in that** it includes converter means of said first and second analogue electrical signals into digital signals, and
   **in that** said processing means include a digital filter arrangement applied to the ambient noise digital signal, having a adaptive transfer function, adapted to output an adapted noise signal, and are arranged to determine a target auscultation signal by subtraction of the filtered digital ambient noise signal from the digital auscultation signal, the filter coefficients of said adaptive digital filter arrangement being calculated according to the minimisation of the energy of the target auscultation signal.

2. Device according to claim 1, wherein said processing means are arranged to calculate the filter coefficients of said digital filter arrangement according to the minimisation of an energy functional $\Phi^2$ defined as:

$$\Phi^2 = \sum_{n=-M}^{0} S(n)^2 + \sum_{k=0}^{N-1} X^2(k)$$

in which S(n) is the target auscultation signal and *X(k)* is the impulse response of the filter.

3. Device according to claim 1 or 2, wherein said processing means comprise a delay block adapted to delay the application of the auscultation signal in input to said filter arrangement.

4. Device according to any one of the previous claims, wherein said processing means are arranged to suspend the calculation of the filter coefficients of said digital filter arrangement in the case in which the transfer function of said filter arrangement is not correlated to the form previously assumed.

5. Device according to any one of the previous claims, comprising frequency equaliser means adapted to carry out frequency equalisation of the target auscultation signal in view of the representation as digital datum or acoustic signal.

6. Device according to any one of the previous claims, comprising a pair of microphone transducers arranged at an auscultation capsule of the device, respectively a first microphone transducer arranged in communication with the auscultation chamber of said capsule and a second microphone transducer, arranged in communication with an outer region adjacent to the auscultation chamber, without acoustic connection with it.

7. Device according to any one of the previous claims, including electro-acoustic transducer means for the acoustic representation of the determined target auscultation signal.

8. Device according to any one of the previous claims, including display means for visually representing the determined target auscultation signal.

9. Device according to any one of the previous claims, including means of communication of the determined target auscultation signal to external units.

10. Device according to any one of the previous claims, including storage means of the determined target auscultation signal.

11. Method of auscultation of heart or lung sounds or similar acoustic signals generated by the internal organs of a body, comprising:

- the acquisition, through a first acoustic channel, of an auscultation signal including a useful signal superimposed to an ambient noise signal, and the generation of a first analogue electrical signal indicative of said auscultation signal;
- the acquisition, through un second acoustic channel in proximity to said first channel, of an ambient noise signal only, and the generation of a second analogue electrical signal indicative of said ambient noise signal; and
- the rejection of the ambient noise signal component from said auscultation signal,

**characterised in that** it includes:

- the conversion of said first and second analogue electrical signals into digital signals;
- the digital filtering of said ambient noise digital signal, according to an adaptive transfer function, to output an adapted noise signal; and
- the calculation of a target auscultation signal by subtraction of the filtered digital ambient noise signal from the digital auscultation signal, the filter coefficients of said adaptive digital filter arrangement being calculated according to the minimisation of the energy of the target auscultation signal.

12. Method according to claim 11, comprising the calculation of the filter coefficients of said digital filter arrangement according to the minimisation of an energy functional $\Phi^2$ defined as:

$$\Phi^2 = \sum_{n=-M}^{0} S(n)^2 + \sum_{k=0}^{N-1} X^2(k)$$

in which S(n) is the target auscultation signal and *X(k)* is the filter impulse response.

13. Method according to claim 11 or 12, wherein the application of the auscultation signal in input to said filter arrangement is delayed by a predetermined number of samples.

**14.** Method according to any one of claims 11 to 13, wherein the calculation of the filter coefficients of said digital filter arrangement is suspended in the case in which the transfer function of said filter arrangement is not correlated to the form previously assumed.

**15.** Method according to any one of claims 11 to 14, comprising a frequency equalisation of the target auscultation signal in view of its representation as digital datum or acoustic signal.

**16.** Method according to any one of claims 11 to 15, including the acoustic representation of the determined target auscultation signal.

**17.** Method according to any one of claims 11 to 16, including the visual representation of the determined target auscultation signal.

**18.** Method according to any one of claims 11 to 17, including the communication of the determined target auscultation signal to external units.

**19.** Method according to any one of claims 11 to 18, including the storage of the determined target auscultation signal.

# FIG.1

# FIG.2

# FIG.3

# FIG.4a

# FIG.4b

# FIG.4c

# FIG.4d

# FIG.5a

# FIG.5b

# FIG.5c

# FIG.5d

# FIG.6

# FIG.7

**European Patent Office**

# EUROPEAN SEARCH REPORT

Application Number

EP 07 42 5340

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 467 775 A (CALLAHAN THOMAS F [US] ET AL) 21 November 1995 (1995-11-21)<br>* the whole document *<br>----- | 1-19 | INV.<br>A61B7/00<br>A61B7/04 |
| X | PATEL SAMIR B ET AL: "An adaptive noise reduction stethoscope for auscultation in high noise environments"<br>JOURNAL OF THE ACOUSTICAL SOCIETY OF AMERICA, AIP / ACOUSTICAL SOCIETY OF AMERICA, MELVILLE, NY, US,<br>vol. 103, no. 5, May 1998 (1998-05), pages 2483-2491, XP012000255<br>ISSN: 0001-4966<br>See sections "I.STETHOSCOPE COUPLER", "II. ADAPTIVE NOISE CANCELLATION",<br>"III.EXPERIMENTAL APPARATUS AND PROCEDURE".<br>See figures 1 and 3.<br>----- | 1-19 | |
| X | US 5 812 678 A (SCALISE STANLEY J [US] ET AL) 22 September 1998 (1998-09-22)<br>* column 7, line 57 - column 8, line 53; figure 2 *<br>* column 15, line 48 - column 16, line 39 *<br>----- | 1-19 | TECHNICAL FIELDS SEARCHED (IPC)<br><br>A61B |
| X | SUZUKI A ET AL: "REAL-TIME ADAPTIVE CANCELLING OF AMBIENT NOISE IN LUNG SOUND MEASUREMENT"<br>MEDICAL AND BIOLOGICAL ENGINEERING AND COMPUTING, SPRINGER, HEILDELBERG, DE,<br>vol. 33, no. 5,<br>1 September 1995 (1995-09-01), pages 704-708, XP000530860<br>ISSN: 0140-0118<br>See sections "1. Introduction", "2. Theory", "3.1. Method".<br>----- | 1-7,<br>11-16 | |

The present search report has been drawn up for all claims

| Place of search | Date of completion of the search | Examiner |
|---|---|---|
| The Hague | 6 December 2007 | Dhervé, Gwenaëlle |

EPO FORM 1503 03.82 (P04C01)

**ANNEX TO THE EUROPEAN SEARCH REPORT
ON EUROPEAN PATENT APPLICATION NO.**

EP 07 42 5340

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2007

| Patent document cited in search report | | Publication date | Patent family member(s) | | Publication date |
|---|---|---|---|---|---|
| US 5467775 | A | 21-11-1995 | AU<br>WO | 5313196 A<br>9629009 A1 | 08-10-1996<br>26-09-1996 |
| US 5812678 | A | 22-09-1998 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040037429 A